# EUROPÄISCHE PATENTANMELDUNG

(11) **EP 1 154 051 A1**
(43) Veröffentlichungstag der Anmeldung: **14.11.2001**
(21) Anmeldenummer: 01110994.9
(22) Anmeldetag: 07.05.2001
(51) Int. Cl.: D01G 9/08, A61F 13/15

(54) **Vorrichtung und Verfahren zum Herstellen von Saugkissen aus einem Flocken-Luft-Gemisch**

(30) Priorität: 09.05.2000 DE 10022500
(71) Anmelder: Winkler + Dünnebier Aktiengesellschaft, 56562 Neuwied (DE)
(72) Erfinder: Geisen, Armin, 56581 Melsbach (DE); Haase, Sascha, 56564 Neuwied (DE); Mertgen, Ulrich, 56584 Meinborn (DE)
(74) Vertreter: Strych, Werner Maximilian Josef, Dr.

(57) **Zusammenfassung**

Es wird eine Vorrichtung bzw. ein Verfahren zum Herstellen von Saugkissen (2) aus einem Flocken-Luft-Gemisch, welches durch eine Rohrleitung (7) einem Formvertiefungsträger (11) zur Bildung des Saugkissens zugeführt wird, wobei sich der Formvertiefungsträger (11) in einer vorgegebenen Drehrichtung dreht oder in einer vorgegebenen Umlaufrichtung umläuft, beschrieben, welche bzw. welches überschüssige Luft im Bereich der Flockenlegeeinrichtung (4) auf einfache Weise abgsaugen soll, ohne den Prozeß des Formens der Saugkissen zu beeinträchtigen. Zur Lösung dieser Aufgabe wird vorgeschlagen, die Rohrleitung (7) in wenigstens zwei Leitungen (7',7'') zu verzweigen, so dass wenigstens eine erste Leitung (7'), durch welche ein erster Teilstrom des Flocken-Luft-Gemisches strömt, und eine zweite Leitung (7''), durch welche ein zweiter Teilstrom des Flocken-Luft-Gemisches strömt, entstehen, und die erste Leitung (7') sowie die zweite Leitung (7'') derart auf den Formvertiefungsträger (11) zu richten, dass der zweite Teilstrom in Drehrichtung oder in Umlaufrichtung betrachtet im wesentlichen hinter dem ersten Teilstrom auf den Formvertiefungsträger trifft.

## Beschreibung

Die vorliegende Erfindung betrifft eine Vorrichtung bzw. ein Verfahren zum Herstellen von Saugkissen aus einem Flocken-Luft-Gemisch gemäß dem Oberbegriff des Anspruchs 1 bzw. des Anspruchs 15.

Bei Maschinen zur Herstellung von saugfähigen Kissen für Damenbinden, Höschenwindeln und ähnliches ist es üblich, auf einer Flockenlegeeinrichtung ein Saugkissen oder Flockenkern aus einem hydrophilen Flockengemisch zu bilden. Die hierzu benötigten Flocken werden der Flockenlegeeinrichtung als Flocken-Luft-Gemisch von einer Zerfaserungseinrichtung kommend durch eine Rohrleitung zugeführt.

Da die Zerfaserungseinrichtung oder Mühle während des Betriebs große Mengen an mechanischer Energie in Wärmeenergie umsetzt, heizt sie sich stark auf. Dieser Umstand erfordert einen möglichst großen, von der Zerfaserungseinrichtung abgehenden Tragluftstrom für den Transport des Flocken-Luft-Gemisches, welcher die gewünschte Wärmeabfuhr von der Zerfaserungseinrichtung bewirkt und diese somit kühlt. Der verhältnismäßig große Tragluftstrom bringt im Bereich der Flockenlegeeinrichtung den Nachteil mit sich, dass dort zu große Luftmengen anfallen, welche von dem Saugkasten der Flockenlegeeinrichtung nicht schnell genug abgesaugt werden können und daher den Prozeß des Formens der Saugkissen aus dem Flocken-Luft-Gemisch beeinträchtigen.

Es ist daher die Aufgabe der vorliegenden Erfindung, eine Vorrichtung bzw. ein Verfahren zum Herstellen von Saugkissen aus einem Flocken-Luft-Gemisch zu schaffen, bei welcher bzw. bei welchem überschüssige Luft im Bereich der Flockenlegeeinrichtung auf einfache Weise abgesaugt werden kann, ohne den Prozeß des Formens der Saugkissen zu beeinträchtigen.

Diese Aufgabe wird mit einer Vorrichtung mit den Merkmalen des Anspruchs 1 bzw. mit einem Verfahren mit den Merkmalen des Anspruchs 15 gelöst. Weitere Ausgestaltungen der Erfindung ergeben sich aus den Unteransprüchen.

Erfindungsgemäß wird eine Vorrichtung zum Herstellen von Saugkissen aus einem Flocken-Luft-Gemisch, welches durch eine Rohrleitung einem Formvertiefungsträger zur Bildung des Saugkissens zugeführt wird, wobei sich der Formvertiefungsträger in einer vorgegebenen Drehrichtung dreht oder in einer vorgegebenen Umlaufrichtung umläuft, vorgeschlagen, welche dadurch gekennzeichnet ist, dass die Rohrleitung in wenigstens zwei Leitungen verzweigt, so dass wenigstens eine erste Leitung, durch welche ein erster Teilstrom des Flocken-Luft-Gemisches strömt, und eine zweite Leitung, durch welche ein zweiter Teilstrom des Flocken-Luft-Gemisches strömt, entstehen, wobei die erste Leitung und die zweite Leitung derart auf den Formvertiefungsträger gerichtet sind, dass der zweite Teilstrom in Drehrichtung oder in Umlaufrichtung betrachtet im wesentlichen hinter dem ersten Teilstrom auf den Formvertiefungsträger trifft. Bei dem Formvertiefungsträger kann es sich beispielsweise um ein sich drehendes Flockenformrad oder um ein um Führungsrollen umlaufendes, endloses Flockenformband bzw. eine entsprechende Flockenformkette handeln.

Der Formvertiefungsträger ist Bestandteil einer Flockenlegeeinrichtung, welche als weitere wesentliche Komponente einen hohlen Flockenkasten umfaßt, wobei sich der Formvertiefungsträger relativ zu dem Flockenkasten bewegt. Erfindungsgemäß münden in den Flockenkasten wenigstens zwei Leitungen, die jeweils einen Teilstrom des Flocken-Luft-Gemisches transportieren und dem Formvertiefungsträger zuführen.

Aufgrund der Bewegung des Formvertiefungsträgers gegenüber dem Flockenkasten treten kontinuierlich Formvertiefungen in denjenigen Raumbereich ein, welcher von dem Flockenkasten mit Flocken-Luft-Gemisch versorgt wird. Diese Formvertiefungen sind zunächst noch nicht bzw. kaum mit Flocke befüllt, so dass ihre siebartigen Böden noch eine verhältnismäßig gute Luftdurchlässigkeit aufweisen. Genau diesen Umstand macht sich die vorliegende Erfindung dadurch zunutze, dass sie einen der beiden Teilströme des Flocken-Luft-Gemisches in denjenigen Bereich des Flockenkastens leitet, in welchem sich Formvertiefungen mit geringem Flockenfüllgrad und somit gut luftdurchlässigem, siebartigem Boden befinden. Dadurch kann die Saugeinrichtung der Flockenlegeeinrichtung in vorteilhafter Weise größere Luftmengen durch die noch nicht mit Flocke verstopften Böden der Formvertiefungen aus dem Flockenkasten absaugen. Auf diese Weise wird die über den Teilstrom abgezweigte, überschüssige Luft effizient aus dem Flockenkasten entfernt und gleichzeitig die in dem Teilstrom enthaltene Flocke für den Saugkissenaufbau genutzt.

Der Wirkungsgrad der erfindungsgemäßen Luftabfuhr kann noch verbessert werden, wenn die Verzweigung der Rohrleitung so vorgenommen wird, dass der erste Teilstrom einen höheren, volumenspezifischen Flockenanteil und der zweite Teilstrom einen niedrigeren, volumenspezifischen Flockenanteil aufweist. Da der zweite Teilstrom auf eine Stelle des Formvertiefungsträgers gerichtet ist, welche in Dreh- oder Umlaufrichtung betrachtet im wesentlichen hinter dem Auftreffbereich des ersten Teilstroms auf den Formvertiefungsträger liegt, werden die in den Überdeckungsbereich des Flockenkastens neu eintretenden Formvertiefungen in diesem Fall weniger schnell mit Flocke befüllt und ihre siebartigen Böden weisen über einen längeren Zeitraum hinweg eine höhere Luftdurchlässigkeit auf. Diese wiederum ermöglicht ein effizienteres Absaugen der überschüssigen Luft.

Es sei ausdrücklich erwähnt, dass die Teilströme im Rahmen der vorliegenden Erfindung dieselben volumenspezifischen Flockenanteile sowie auch unterschiedliche volumenspezifische Flockenanteile enthalten können.

Erfindungsgemäß können auch drei oder noch mehr Teilströme aus der das Flocken-Fluft-Gemisch zuführenden Rohrleitung abgezweigt werden. Besonders vorteilhaft ist es in diesem Fall, die dritte Leitung derart auszurichten, dass der dritte Teilstrom in Dreh- oder Umlaufrichtung betrachtet im wesentlichen vor dem ersten Teilstrom auf den Formvertiefungsträger trifft. Der erste Teilstrom verläuft dann zwischen dem zweiten und dem dritten Teilstrom und es kann ihm ein hydrophiler Zusatzstoff, üblicherweise ein Super Absorbent Polymer (SAP), zugesetzt werden. Dieser Zusatzstoff kann auf diese Weise gezielt in den mittleren Schichtbereich des fertigen Saugkissens eingebracht werden. Er wird dadurch sicher in dem Saugkissen gehalten und kann sich nicht in unerwünschter Weise aus dem Flockenverbund des Saugkissens lösen.

Zur Auflösung von Flockenverdichtungen bzw. Flockenverklumpungen innerhalb der Flocken-Luft-Gemische der Teilströme, welche durch Rohrreibung, Strömungsturbulenzen sowie statische Aufladung entstehen können, können im Verlauf der die Teilströme führenden Leitungen pneumatische Auflöseeinrichtungen zum Auflösen der Flockenverklumpungen vorgesehen werden, welche die Flockenverklumpungen auflösen, indem sie das Flocken-Luft-Gemisch des jeweiligen Teilstromes samt der darin enthaltenen Flockenverklumpungen derart beschleunigen, dass die Flockenverklumpungen zerplatzen oder zerreißen.

Überraschenderweise hat sich herausgestellt, dass Flockenverklumpungen, welche aus verdichteten Zelluloseflocken bzw. -fasern bestehen, aufgelöst werden können, wenn sie plötzlich ruck-, stoß- oder sprungartig mit Hilfe eines gerichteten Fluidstroms, insbesondere Luftstroms, hoher kinetischer Energie beschleunigt werden. Dabei treten Beschleunigungs- und/oder Fluidreibungskräfte auf, welche die mechanischen und/oder elektrostatischen Adhäsionskräfte, die zwischen den eine Flockenverklumpung bildenden Flocken wirken, ohne weiteres überwinden. Darüber hinaus erzeugt die erfindungsgemäße, pneumatische Auflöseeinrichtung vorzugsweise turbulente Strömungen, deren richtungs- sowie betragsmäßig willkürlich wechselnde Beschleunigungen dazu führen, dass die Flockenverklumpungen zerfallen. Es hat sich als besonders vorteilhaft herausgestellt, das Flocken-Luft-Gemisch auf Überschallgeschwindigkeit , vorzugsweise auf bis zu 2-fache Schallgeschwindigkeit, zu beschleunigen. Derart hohe Geschwindigkeiten gewährleisten, dass in der Auflöseeinrichtung turbulente Strömungen erreicht werden und das Flocken-Luft-Gemisch des jeweiligen Teilstroms hinreichend ruck-, stoß- oder sprungartig beschleunigt werden.

Je nachdem wie groß die eine Flockenverklumpung zusammenhaltenden Kräfte sind, erfolgt die Auflösung der Flockenverklumpung früher oder später. Eine weniger fest zusammengehaltene Flockenverklumpung kann bereits zu Beginn der Beschleunigungsphase auseinandergerissen werden während eine fester zusammengehaltene Flockenverklumpung unter Umständen erst nach Erreichen eines hinreichenden Turbulenzgrades der Strömung des Flocken-Luft-Gemisches zersetzt werden kann. In jedem Fall wird erfindungsgemäß erreicht, dass im Gegensatz zu den bekannten Pralleinrichtungen sämtliche im Flocken-Luft-Gemisch enthaltenen Flockenverklumpungen von den Beschleunigungskräften erfasst werden.

Eine pneumatische Auflöseeinrichtung kann erfindungsgemäß jeweils im Endbereich der den Flocken-Luft-Gemisch-Strom liefernden Leitungen der Teilströme angeordnet werden. Vorzugsweise wird die Auflöseeinrichtung direkt in die Mündung der jeweiligen Leitung in die Flockenlegeeinrichtung eingesetzt. Die Flockenlegeeinrichtung umfaßt ein sich drehendes Flockenformrad sowie einen ortsfesten Flockenkasten, in welchen wenigstens eine der Leitungen über die pneumatische Auflöseeinrichtung mündet. Aufgrund des gerichteten Strahls von im wesentlichen verklumpungsfreiem Flocken-Luft-Gemisch, welcher von der pneumatischen Auflöseeinrichtung erzeugt wird, ist es im Gegensatz zum Stand der Technik sowie in vorteilhafter Weise möglich, diesen Strahl gezielt in eine bestimmte Raumrichtung zu richten. Die in der Umfangsfläche des Flockenformrades befindlichen Formvertiefungen bzw. Mulden zum Ausbilden der Saugkissen können dadurch effektiver mit Flocke befüllt werden.

Erfindungsgemäß kann die pneumatische Auflöseeinrichtung von einer von dem Flocken-Luft-Gemisch umströmten Düse gebildet werden, welche etwa im mittleren Bereich des Querschnitts der Leitung des jeweiligen angeordnet ist. Bei dieser Variante der Auflöseeinrichtung ist es allerdings erforderlich, die mit hohem Druck aus der Düse auszubringende Druckluft ebenso in den mittleren Bereich des Leitungsquerschnitts zu bringen. Dies hat zur Folge, dass wenigstens eine entsprechende Pneumatikleitung in den mittleren Bereich des Leitungsquerschnitts geführt werden muß, was bedeutet, dass die Pneumatikleitungen im Strömungsweg des Flocken-Luft-Gemisches stehen und somit zusätzlich zu der pneumatischen Auflösung der Flockenverklumpungen eine mechanische Auflösungswirkung im Sinne von bereits bekannten Auflöseeinrichtungen in Form von Prallstäben bewirken.

Vorzugsweise wird die pneumatische Auflöseeinrichtung in Form einer rohrstückartigen Injektordüse gewählt, welche selbst von dem Flocken-Luft-Gemisch des jeweiligen Teilstroms durchströmt wird. Über einen, vorzugsweise mehrere Zuführdurchlässe in Form von Zuführkanälen wird dieser Injektordüse ein unter hohem Druck stehendes Druckfluid, insbesondere Druckluft, zugeführt. Dieses Druckfluid kann entweder von einer extra hierfür vorgesehenen Druckfluidquelle oder von einer ohnehin für andere Systeme der hier in Rede stehenden Maschinen bereits vorhandenen Druckfluidquelle stammen. Die vorzugsweise mehreren Zuführkanäle können sich erfindungsgemäß im äußeren Umfangsbereich desjenigen Raumes der Injektordüse befinden, der von dem Flocken-Luft-Gemisch durchströmt wird.

Die pneumatische Auflöseeinrichtung bzw. die Injektordüse wirken fluidmechanisch wie ein Fluid- bzw. Luftstromverstärker. Auf ihrer Eintritts- bzw. Ansaugseite entsteht ein Unterdruck während auf ihrer Austritts- bzw. Ausblasseite ein Überdruck entsteht. Die Auflöseeinrichtung bzw. Injektordüse kann daher wie ein am Ende der den Flokken-Luft-Strom liefernden Leitung des jeweiligen Teilstroms angeordnetes Sauggebläse wirken, so dass je nach Gesamtlänge der Leitungen in vorteilhafter Weise auf ein weiteres Gebläse in der Flockenförderstrecke verzichtet werden kann.

Erfindungsgemäß wird auch ein Verfahren zum Herstellen von Saugkissen aus einem Flocken-Luft-Gemisch, welches durch eine Rohrleitung einem Formvertiefungsträger zur Bildung des Saugkissens zugeführt wird, wobei sich der Formvertiefungsträger in einer vorgegebenen Drehrichtung dreht oder in einer vorgegebenen Umlaufrichtung umläuft, vorgeschlagen, welches dadurch gekennzeichnet ist, dass das Flocken-Luft-Gemisch in wenigstens zwei Teilströme aufgeteilt wird, so dass wenigstens ein erster Teilstrom und ein zweiter Teilstrom entstehen, wobei der zweite Teilstrom derart auf den Formvertiefungsträger gerichtet wird, dass er in Drehrichtung oder Umlaufrichtung betrachtet im wesentlichen hinter dem ersten Teilstrom auf den Formvertiefungsträger trifft.

Nachfolgend wird eine Ausführungsform der vorliegenden Erfindung beispielhaft anhand der beigefügten Zeichnungen beschrieben. Es zeigen:
- Fig. 1:: eine schematische Seitenansicht einer erfindungsgemäßen Vorrichtung zum Herstellen von Saugkissen aus einem Flocken-Luft-Gemisch;
- Fig. 2:: eine pneumatische Auflöseeinrichtung in Form einer Injektordüse;
- Fig. 3: eine Ansicht der Injektordüse gemäß Fig. 2 von oben; und
- Fig. 4:: den Schnitt A-A gemäß Fig. 3.

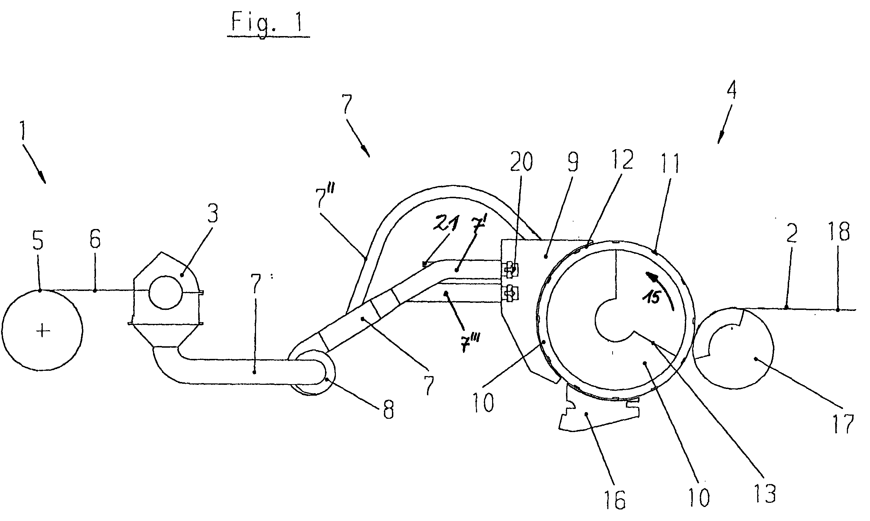

Fig. 1 zeigt eine erfindungsgemäße Vorrichtung zum Herstellen von Saugkissen 2 aus einem Flocken-Luft-Gemisch. Die Saugkissen 2 können Bestandteil von Damenbinden, Höschenwindeln oder ähnlichen Hygieneartikeln sein. Eine Mühle oder Zerfaserungseinrichtung 3 wird von einer Vorratsrolle 5 aus mit einer Papp- bzw. Zellstoffbahn 6 beschickt. Die Zellstoffbahn 6 wird in der Zerfaserungseinrichtung 3 zu Zellstoff-Flocken aufgelöst. Alternativ könnten die Zellstoff-Flocken auch auf sonstige Weise zur Verfügung gestellt werden, beispielsweise bereits als fertige Zellstoff-Flocken vorliegen. Diese Flocken werden mit Tragluft versetzt und als Flocken-Luft-Gemisch durch eine Rohrleitung 7 zu einer Flockenlegeeinrichtung 4 transportiert. Je nach Länge der Rohrleitung 7 kann dabei ein Gebläse bzw. Ventilator 8 zur Unterstützung des Tragluftstromes im Verlauf der Rohrleitung 7 vorgesehen werden.

Die Flockenlegeeinrichtung 4 umfaßt im wesentlichen ein Flockenformrad 10 sowie einen hohlen Flockenkasten 9, der das Flockenformrad 10 in einem vorgegebenen Umfangsbereich überdeckt und dort zum Flockenformrad 10 hin offen ist. Das Flokkenformrad 10 dreht sich gemäß Drehrichtung 15 entgegen dem Uhrzeigersinn. Das Flockenformrad 10 weist in seinem Mantel bzw. in seiner äußeren Umfangsfläche Mulden oder Formvertiefungen 11 auf, welche mit siebartigen Böden 12 versehen sind. Ein ortsfester, d.h. sich nicht mit dem Flockenformrad 10 mitdrehender Saugkasten 13 ermöglicht es, Flocken-Luft-Gemisch in demjenigen Umfangsbereich des Flockenformrades 10, welcher von dem Flockenkasten 9 überdeckt wird, durch die siebartigen Böden 12 in die Formvertiefungen 11 zu saugen. Dadurch entstehen in denjenigen Formvertiefungen 11, welche sich in dem vom Flockenkasten 9 überdeckten Bereich befinden, die gewünschten Flockenkerne bzw. Saugkissen 2.

In Drehrichtung 15 vor dem Flockenformrad 10 ist eine Abbürsteinrichtung 16 angeordnet, welche das über die äußere Umfangsfläche aus den Formvertiefungen 11 hinausragende Flockenmaterial entfernt und über eine nicht dargestellte Rohrleitung zur Zerfaserungseinrichtung 3 zurückführt. Anschließend werden die auf diese Weise bündig gebürsteten Saugkissen mittels einer Saugwalze 17 aus den Formvertiefungen 11 entnommen und auf ein Transportband 18 gelegt, welches sie einer nicht gezeigten Maschine zur Herstellung von Damenbinden, Slipeinlagen, Höschenwindeln oder ähnliches zuführt.

Wie in Fig. 1 zu erkennen ist, verzweigt die Rohrleitung 7 kurz hinter dem Gebläse 8 an einer ersten Verzweigungsstelle. An dieser Stelle zweigt von der als Hauptstromleitung fungierenden ersten Leitung 7' die als Nebenstromleitung fungierende zweite Leitung 7" ab. Die zweite Leitung 7" verläuft bogenförmig und mündet von schräg oben in den Flockenkasten 9. Da der die Leitung 7' durchströmende erste Teilstrom an der ersten Verzweigungsstelle nicht umgelenkt wird, weist er bei der gezeigten Ausführungsform einen höheren volumenspezifischen Flockenanteil auf als der die Leitung 7" durchströmende zweite Teilstrom, welcher im wesentlichen überschüssige Tragluft in den Flockenkasten 9 befördert. Durch Variieren der Geometrie der ersten Verzweigungsstelle sowie der dortigen Strömungsführung und des Querschnittsverhältnisses der ersten Leitung 7' zu der zweiten Leitung 7" können die gewünschten Verhältnisse des ersten Teilstroms zu dem zweiten Teilstrom sowie deren Flockenanteile eingestellt werden. Beispielsweise ist denkbar, an der ersten Verzweigungsstelle ein Ventil vorzusehen, mit welchem die zweite Leitung 7" oder die erste Leitung 7' bei Bedarf abgesperrt werden kann.

Die Richtung, in welcher die zweite Leitung 7" in den Flockenkasten 9 mündet, wurde so gewählt, dass der zweite Teilstrom in denjenigen Bereich des Flockenkastens 9 geführt wird, in welchen noch unbefüllte Formvertiefungen 11 in Drehrichtung 15 des Flockenformrades 10 einlaufen. Der Saugkasten 13 kann die überschüssige Luft in Form des zweiten Teilstromes somit ohne großen Strömungswiderstand durch die siebartigen Böden 12 der Formvertiefungen 11 absaugen.

Durch die erste Leitung 7' gelangt der Hauptstrom des Flocken-Luft-Gemisches über eine pneumatische Auflöseeinrichtung 20 zum Auflösen von Flockenverklumpungen in den Flockenkasten 9. Die einen gerichteten Luftstrahl erzeugende Auflöseeinrichtung 20 ist dabei so ausgerichtet, dass der Hauptstrom in Drehrichtung 15 betrachtet vor dem Nebenstrom aus der Leitung 7" auf das Flockenformrad 10 trifft.

Stromabwärts der ersten Verzweigungsstelle befindet sich eine zweite Verzweigungsstelle, an welcher die Hauptstromleitung bzw. die erste Leitung 7' in eine dritte Leitung 7"' verzweigt. Die dritte Leitung 7"' verläuft unterhalb der Leitung 7' und mündet ebenso unterhalb dieser in den Flockenkasten 9. Auch im Mündungsbereich der Leitung 7"' ist eine pneumatische Auflöseeinrichtung 20 zum Auflösen von Flockenverklumpungen vorgesehen. Wie zu erkennen ist, wird der dritte Teilstrom an der zweiten Verzweigungsstelle im Gegensatz zu dem Hauptstrom umgelenkt, so dass der dritte Teilstrom bei der gezeigten Ausführungsform einen geringeren volumenspezifischen Flockenanteil enthält als der Hauptstrom. Auch an der zweiten Verzweigungsstelle kann bei Bedarf ein Ventil zum Absperren der ersten Leitung 7' oder der dritten Leitung 7"' vorgesehen werden.

Stromabwärts der zweiten Verzweigungsstelle ist an der ersten Leitung 7' ein Anschlußstutzen 21 zum Anschließen einer nicht gezeigten Zufuhrleitung zum Zuführen eines hydrophilen Zusatzstoffes vorgesehen. Bei diesem hydrophilen Zusatzstoff handelt es sich beispielsweise um ein Super Absorbent Polymer (SAP).,Durch das Verzweigen der Rohrleitung 7 in die drei Leitungen 7', 7" und 7"' kann das SAP in geschickter Weise in die mittlere Schicht der fertigen Saugkissen 2 eingebunden werden. Dies wird dadurch erreicht, dass die leeren Formvertiefungen 11, welche in Drehrichtung 15 in den Überdeckungsbereich zwischen Flockenkasten 9 und Flokkenformrad 10 einlaufen, zunächst mittels des zweiten Teilstroms aus der zweiten Leitung 7" mit Flocke befüllt werden. An den siebartigen Böden der Formvertiefungen 11 bildet sich dementsprechend eine erste Schicht des Saugkissens 2. Mit zunehmender Drehung nähert sich die betreffende Formvertiefung 11 immer mehr dem aus der ersten Leitung 7' austretenden, ersten Teilstrom, welcher sich aus einem Flokken-Luft-SAP-Gemisch zusammensetzt. Aus diesem Gemisch bildet sich in der Formvertiefung 11 eine weitere, zweite Schicht des Saugkissens 2. Durch weiteres Drehen in Drehrichtung 15 nähert sich die Formvertiefung 11 derjenigen Stelle, auf welche der dritte Teilstrom aus der dritten Leitung 7"' gerichtet ist. Da der dritte Teilstrom kein SAP enthält, entsteht in der Formvertiefung 11 dadurch eine dritte Schicht des Saugkissens 2, welche diesen Zusatzstoff ebenso wie die zuerst ausgebildete Schicht nicht enthält.

Der hydrophile Zusatzstoff SAP wird auf diese Weise gewissermaßen in dem Saugkissen 2 verpackt, wodurch gewährleistet wird, dass sich das SAP nicht ohne weiteres aus dem Saugkissen 2 herauslösen und mit Schleimhäuten in Berührung kommen kann.

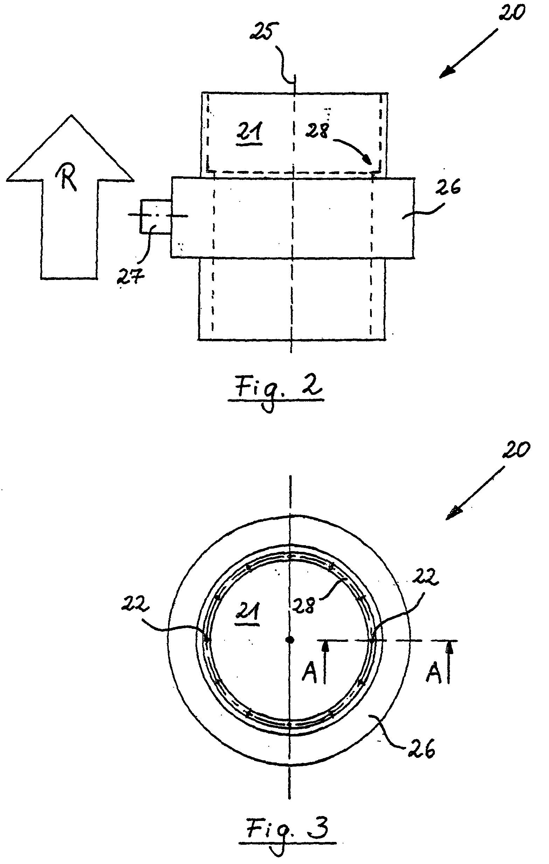

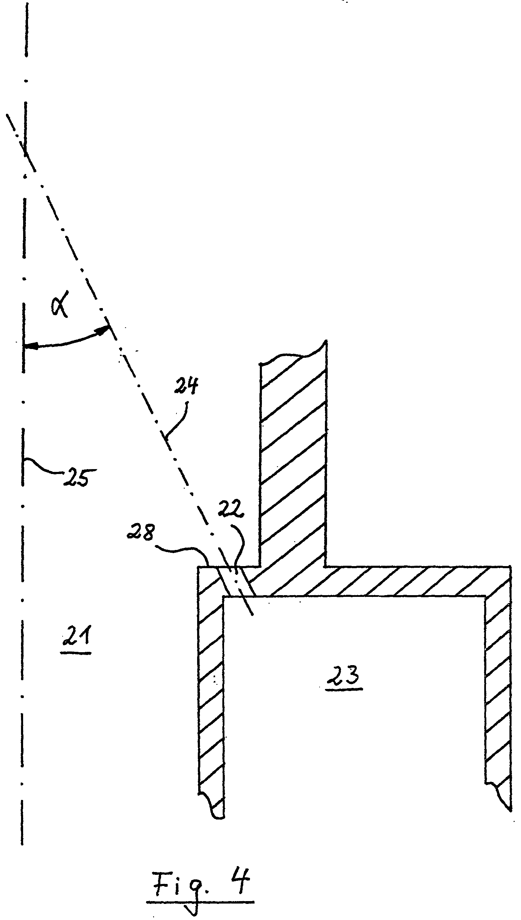

In den Fig. 2 und 3 ist eine als pneumatische Auflöseeinrichtung fungierende Injektordüse 20 gezeigt. Sie besteht aus einem verhältnismäßig kurzen Rohrstück, welches in das stromabwärtige Ende der Leitungen 7' und 7"' eingesetzt wird. Die zweite Leitung 7" mündet bei der gezeigten Ausführungsform ohne Injektordüse in den Flockenkasten 9, da der Flockenanteil in dem zweiten Teilstrom so gering ist, dass mit der Entstehung nennenswerter Flockenverklumpungen in der Leitung 7" nicht zu rechnen ist. Die Injektordüse 20 weist in ihrem in Längsrichtung mittleren Bereich einen Wulst 26 auf, in welchem ein vollständig umlaufender Ringkanal 23 ausgebildet ist, wie in der Schnittdarstellung gemäß Fig. 4 zu erkennen ist. Der Ringkanal 23 steht über den Anschlußstutzen 27 in Strömungsverbindung mit einer nicht gezeigten Pneumatik- bzw. Druckluftquelle, welche eigens für die Injektordüse 20 vorgesehen werden kann oder bereits für andere Systeme der jeweiligen Maschine vorhanden ist.

In der in Fig. 2 gezeigten Darstellung durchströmt das Flocken-Luft-Gemisch gemäß Pfeil R die Injektordüse 20 von unten nach oben. Dabei durchströmt es insbesondere den Durchströmungsraum 21, welcher bei der gezeigten Ausführungsform einen zylindrischen Querschnitt aufweist, alternativ jedoch auch mit einem in Durchströmungsrichtung R zunehmenden Querschnitt versehen sein kann und dadurch als Diffusor wirkt. Wie den Fig. 2 und 3 zu entnehmen ist, weist der Durchströmungsraum 21 einen größeren Innendurchmesser auf als der restliche, in Fig. 2 darunter liegende Raum der Injektordüse 20. Dadurch entsteht auf Höhe der unteren Begrenzungsebene des Durchströmungsraums 21 ein kreisringförmiger Absatz 28, wie in der Ansicht gemäß Fig. 3 dargestellt.

Wie am besten in den Fig. 3 und 4 zu sehen ist, münden in die kreisringförmige Fläche des Absatzes 28 bei der gezeigten Ausführungsform insgesamt zwölf als Zuführdurchlässe fungierende Zuführkanäle 22, welche in gleichmäßigen Winkelabständen über den Verlauf des Absatzes 28 verteilt sind. Alternativ ist auch denkbar, weniger oder mehr als zwölf Zuführkanäle vorzusehen oder die Zuführkanäle in ungleichmäßigen Winkelabständen anzuordnen, um bei Bedarf spezielle Strömungsprofile über den Querschnitt der Injektordüse 20 zu erhalten.

Wie in Fig. 4 gezeigt, stehen die Zuführkanäle 22 in Strömungsverbindung mit dem Ringkanal 23 und daher auch mit der an dem Anschlußstutzen 27 angeschlossenen Druckluftquelle.

Gemäß Fig. 4 ist die Achse 24 des Zuführkanals 22 um den Neigungswinkel α gegenüber der Längs- bzw. Symmetrieachse 25 der Injektordüse 20 geneigt. Dadurch wird bewirkt, dass die Druckluft nicht nur axial in Durchströmungsrichtung R, sondern auch mit einer radial nach innen auf die Längsachse 25 gerichteten Geschwindigkeitskomponente zugeführt wird. Hierdurch kann zusätzlich zu den die Flockenverklumpungen auflösenden Beschleunigungskräften noch ein weiterer, die Flockenverklumpungsauflösung unterstützender Effekt bewirkt werden, der darin besteht, dass vorhandene Flockenverklumpungen aus den radial äußeren Bereichen des Strahlquerschnitts radial nach innen geblasen werden und dadurch unter Umständen miteinander kollidieren sowie dabei zerrissen bzw. aufgelöst werden. Der Neigungswinkel α kann erfindungsgemäß 50° oder weniger betragen, wobei für ihn vorzugsweise die Ungleichung 5° ≤ α ≤ 30° gilt. Alternativ kann der Neigungswinkel α auch 0° betragen, was bedeutet, dass die Achse 24 der Zuführkanäle 22 parallel zur Längsachse 25 der Injektordüse 20 verläuft.

Anstatt mehrerer Zuführdurchlässe 22 in Form von Bohrungskanälen kann zwischen Ringkanal 23 und Durchströmungsraum 21 auch ein einziger Zuführdurchlass in Form eines umlaufenden Ringspaltkanals vorgesehen werden. In diesem Fall steht der zuzuführenden Druckluft eine größere Querschnittsfläche zum Einströmen in den Durchströmungsraum 21 zur Verfügung, so dass dem Durchströmungsraum 21 ein größerer und räumlich besser verteilter Volumenstrom an Druckluft zugeführt werden kann und somit eine höhere Effektivität bei der Auflösung der Flockenverklumpungen realisierbar ist.

## Patentansprüche

1. Vorrichtung zum Herstellen von Saugkissen (2) aus einem Flocken-Luft-Gemisch, welches durch eine Rohrleitung (7) einem Formvertiefungsträger (10) zur Bildung des Saugkissens (2) zugeführt wird, wobei sich der Formvertiefungsträger (10) in einer vorgegebenen Drehrichtung (15) dreht oder in einer vorgegebenen Umlaufrichtung umläuft,
**dadurch gekennzeichnet, dass**
die Rohrleitung (7) in wenigstens zwei Leitungen verzweigt, so dass wenigstens eine erste Leitung (7'), durch welche ein erster Teilstrom des Flocken-Luft-Gemisches strömt, und eine zweite Leitung (7"), durch welche ein zweiter Teilstrom des Flocken-Luft-Gemisches strömt, entstehen, wobei die erste Leitung (7') und die zweite Leitung (7") derart auf den Formvertiefungsträger (10) gerichtet sind, dass der zweite Teilstrom in Drehrichtung (15) oder in Umlaufrichtung betrachtet im wesentlichen hinter dem ersten Teilstrom auf den Formvertiefungsträger (10) trifft.

2. Vorrichtung nach Anspruch 1,
**dadurch gekennzeichnet, dass**
die Verzweigung derart ausgebildet ist, dass der erste Teilstrom einen höheren Flokkenanteil und der zweite Teilstrom einen niedrigeren Flockenanteil aufweist.

3. Vorrichtung nach Anspruch 1 oder 2,
**dadurch gekennzeichnet, dass**
eine dritte Leitung (7''') vorgesehen ist, durch welche ein dritter Teilstrom des Flokken-Luft-Gemisches strömt.

4. Vorrichtung nach Anspruch 3,
**dadurch gekennzeichnet, dass**
die dritte Leitung (7"') derart ausgerichtet ist, dass der dritte Teilstrom in Drehrichtung (15) oder in Umlaufrichtung betrachtet im wesentlichen vor dem ersten Teilstrom auf den Formvertiefungsträger (10) trifft, und dem ersten Teilstrom ein hydrophiler Zusatzstoff zuführbar ist, so dass der Zusatzstoff im wesentlichen in einer mittleren Schicht des Saugkissens (2) zu liegen kommt.

5. Vorrichtung nach einem der vorangehenden Ansprüche,
**dadurch gekennzeichnet, dass**
im Verlauf wenigstens einer der Leitungen (7', 7", 7''') eine pneumatische Auflöseeinrichtung (20) zum Auflösen von Flockenverklumpungen innerhalb des jeweiligen Teilstromes vorgesehen ist, welche die Flockenverklumpungen auflöst, indem sie das Flocken-Luft-Gemisch des jeweiligen Teilstromes samt der darin enthaltenen Flockenverklumpungen derart beschleunigt, dass die Flockenverklumpungen zerplatzen oder zerreißen.

6. Vorrichtung nach Anspruch 5,
**dadurch gekennzeichnet, dass**
die Auflöseeinrichtung (20) im stromabwärtigen Endbereich der wenigstens einen Leitung (7', 7", 7''') angeordnet ist.

7. Vorrichtung nach Anspruch 5 oder 6,
**dadurch gekennzeichnet, dass**
die Auflöseeinrichtung eine im wesentlichen im mittleren Bereich des Querschnitts der wenigstens einen Leitung (7', 7", 7''') angeordnete Düse ist, welche von dem Flocken-Luft-Gemisch umströmt wird und wenigstens einen Düsenkanal aufweist, durch welchen Druckfluid zum Beschleunigen der Flockenverklumpungen von einer Druckfluidquelle zuführbar ist.

8. Vorrichtung nach Anspruch 5 oder 6,
**dadurch gekennzeichnet, dass**
die Auflöseeinrichtung von einer rohrstückartigen Injektordüse (20) gebildet wird, welche einen Durchströmungsraum (21), der von dem Flocken-Luft-Gemisch des jeweiligen Teilstromes durchströmt wird, aufweist und mit wenigstens einem Zuführdurchlaß (22) versehen ist, welcher sich im äußeren Umfangsbereich des Durchströmungsraum (21) befindet und durch welchen dem Durchströmungsraum (21) Druckfluid zum Beschleunigen der Flockenverklumpungen von einer Druckfluidquelle zuführbar ist.

9. Vorrichtung nach Anspruch 8,
**dadurch gekennzeichnet, dass**
der wenigstens eine Zuführdurchlaß (22) mit einem Ringkanal (23) in Strömungsverbindung steht, der mit dem Druckfluid versorgbar ist.

10. Vorrichtung nach Anspruch 9,
**dadurch gekennzeichnet, dass**
mehrere Zuführdurchlässe in Form von Zuführkanälen (22) vorgesehen sind, welche in gleichmäßigen Winkelabständen in dem äußeren Umfangsbereich des Durchströmungsraums (21) angeordnet sind.

11. Vorrichtung nach einem der Ansprüche 8,9 oder 10,
**dadurch gekennzeichnet, dass**
die Achse bzw. die Achsen (24) des Zuführdurchlasses bzw. der Zuführdurchlässe (22) parallel zu der Längsachse (25) der Injektordüse (20) verläuft bzw. verlaufen, so dass das Druckfluid dem Durchströmungsraum (21) im wesentlichen axial in Durchströmungsrichtung (R) des Flocken-Luft-Gemisches des jeweiligen Teilstromes zugeführt wird.

12. Vorrichtung nach einem der Ansprüche 8, 9 oder 10,
**dadurch gekennzeichnet, dass**
die Achse bzw. Achsen (24) des Zuführdurchlasses bzw. der Zuführdurchlässe (22) um einen Neigungswinkel (α) geneigt zu der Längsachse (25) der Injektordüse (20) verläuft bzw. verlaufen, so dass das Druckfluid dem Durchströmungsraum (21) sowohl mit axialer als auch mit radialer Strömungskomponente zugeführt wird.

13. Vorrichtung nach Anspruch 12,
**dadurch gekennzeichnet, dass**
für den Neigungswinkel (α) die Ungleichung 0 ≤ α ≤ 50° gilt.

14. Vorrichtung nach Anspruch 13,
**dadurch gekennzeichnet, dass**
für den Neigungswinkel (α) die Ungleichung 5°≤ α ≤ 30° gilt.

15. Verfahren zum Herstellen von Saugkissen (2) aus einem Flocken-Luft-Gemisch, welches durch eine Rohrleitung (2) einem Formvertiefungsträger (10) zur Bildung des Saugkissens (2) zugeführt wird, wobei sich der Formvertiefungsträger (10) in einer vorgegebenen Drehrichtung (15) dreht oder in einer vorgegebenen Umlaufrichtung umläuft,
**dadurch gekennzeichnet, dass**
das Flocken-Luft-Gemisch in wenigstens zwei Teilströme aufgeteilt wird, so dass wenigstens ein erster Teilstrom und ein zweiter Teilstrom entstehen, wobei der zweite Teilstrom derart auf den Formvertiefungsträger (10) gerichtet wird, dass er in Drehrichtung (15) oder Umlaufrichtung betrachtet im wesentlichen hinter dem ersten Teilstrom auf den Formvertiefungsträger (10) trifft.

16. Verfahren nach Anspruch 15,
**dadurch gekennzeichnet, dass**
die Aufteilung des Flocken-Luft-Gemisches derart erfolgt, dass der erste Teilstrom einen höheren Flockenanteil und der zweite Teilstrom einen niedrigeren Flockenanteil aufweist.

17. Verfahren nach Anspruch 15 oder 16,
**dadurch gekennzeichnet, dass**
das Flocken-Luft-Gemisch in drei Teilströme aufgeteilt wird, so dass ein dritter Teilstrom entsteht.

18. Verfahren nach Anspruch 17,
**dadurch gekennzeichnet, dass**
der dritte Teilstrom derart auf den Formvertiefungsträger (10) gerichtet wird, dass er in Drehrichtung (15) oder in Umlaufrichtung betrachtet im wesentlichen vor dem ersten Teilstrom auf den Formvertiefungsträger (10) trifft, und dem ersten Teilstrom ein hydrophiler Zusatzstoff zugesetzt wird, so dass der Zusatzstoff im wesentlichen in einer mittleren Schicht des Saugkissens (2) zu liegen kommt.
